# FASCICULE DE BREVET EUROPEEN

(11) **EP 2 413 779 B1**
(45) Date de publication et mention de la délivrance du brevet: **16.09.2020**
(21) Numéro de dépôt: 10717700.8
(22) Date de dépôt: 31.03.2010
(51) Int. Cl.: A61B 3/113, A61B 5/16

(54) **SYSTEME DE MISE EN EVIDENCE D'ANOMALIES OCULOMOTRICES**
SYSTEM ZUM NACHWEIS VON OKULOMOTORISCHEN AUFFÄLLIGKEITEN
SYSTEM FOR DEMONSTRATING OCULOMOTOR ABNORMALITIES

(30) Priorité: 01.04.2009 FR 0952102
(43) Date de publication de la demande: 08.02.2012
(73) Titulaire: Suricog, 75015 Paris (FR)
(72) Inventeur: KINKINGNEHUN, Serge, F-94400 Vitry Sur Seine (FR); MAILLARD, Mickael, F-92800 Puteaux (FR); ARGUDO, Mathieu, F-64700 Hendaye (FR)
(74) Mandataire: Nony
(86) Numéro de dépôt international: PCT/FR2010/050610
(87) Numéro de publication internationale: WO 2010/112771

(56) Documents cités:
- FR-A- 2 593 381
- JP-A- 2003 319 907
- US-A1- 2008 058 681
- US-B1- 6 231 187
- VAN STOCKUM S ET AL: "Don't look now or look away: Two sources of saccadic disinhibition in Parkinson's disease?" NEUROPSYCHOLOGIA, PERGAMON PRESS, OXFORD, GB, vol. 46, no. 13, 1 novembre 2008 (2008-11-01), pages 3108-3115, XP026034896 ISSN: 0028-3932 [extrait le 2008-07-12]

## Description

L'invention porte sur un système de mise en évidence d'anomalies oculomotrices chez un sujet humain.

Le domaine de l'invention est le domaine de la détection et de la mise en évidence d'anomalies oculomotrices chez un sujet humain et plus particulièrement les anomalies oculomotrices liées à des maladies neurologiques, psychiatriques et neurodéveloppementales, ainsi que l'efficacité et le suivi des soins.

Dans le cas des maladies neurodégénératives, le vieillissement de la population en France fait croître de façon significative le nombre de patients atteints par ces pathologies. La maladie de Parkinson est la deuxième maladie neurodégénérative en France avec plus de 100 000 cas et 10 000 nouveaux cas par an.

Le terme «syndrome parkinsonien » regroupe la maladie de Parkinson et les pathologies neurologiques dont les symptômes, à un stade précoce de la maladie, sont similaires à la maladie de Parkinson.

Les études menées sur plus de 6000 patients atteints de maladies neurologiques ont permis d'établir un lien entre les syndromes parkinsoniens et certaines anomalies oculomotrices, mesurables grâce à des tests appropriés.

Actuellement, les syndromes parkinsoniens sont difficilement différenciables. Un collège de neurologues, dans un centre d'examen neurologique spécialisé, est capable de faire une distinction entre certaines de ces pathologies, en utilisant des batteries de tests approfondis et en ayant généralement recours à plusieurs examens complémentaires, dont l'IRM et la scintigraphie. Cela suppose l'hospitalisation du patient plusieurs jours, avec un temps de diagnostic de plusieurs mois, car l'attente pour un examen IRM peut durer jusqu'à 6 mois et un nouveau rendez-vous avec un spécialiste encore plusieurs mois.

Enfin, un service IRM ou un laboratoire coûtent cher et mobilisent plusieurs opérateurs, ce qui rend ce type de diagnostic inaccessible pour un neurologue libéral.

Actuellement, il n'existe aucun appareil permettant de réaliser une mise en évidence d'anomalies oculomotrices liées aux syndromes parkinsoniens et plus généralement aux maladies neurologiques, psychiatriques ou neurodéveloppementales chez un eucaryote, ainsi que d'évaluer l'effet des soins et leur suivi. FR 2 593 381 A, JP 2003 319907 A, US 6 231 187 B1, US 2008/058681 A1 divulguent des systèmes de détection d'anomalies oculomotrices. Le système, objet de l'invention, constitue une plateforme technologique comparable à un test compagnon

Un but de la présente invention est de remédier aux inconvénients indiqués supra.

Un autre but de la présente invention est de proposer un système et un procédé automatisé de détection ou de mise en évidence d'anomalies oculomotrices plus efficaces que les systèmes et procédés de l'état de la technique.

Il est encore un but de l'invention de proposer un système permettant d'assister un neurologue, un psychiatre ou un spécialiste pour la mise en évidence d'anomalies oculomotrices.

Encore un autre but de l'invention est de proposer un système de mise en évidence d'anomalies oculomotrices permettent une mise en évidence d'anomalies plus rapide.

Enfin, un but de l'invention est de proposer un système permettant de réaliser une mise en évidence d'anomalies oculomotrices moins onéreuses que les pratiques actuelles.

L'invention permet d'atteindre les buts précités par un système de mise en évidence d'anomalies oculomotrices chez un sujet humain comme défini dans les revendications.

Le système selon l'invention permet de déterminer de manière totalement automatisé des anomalies oculomotrices en analysant les mouvements oculaires qui sont provoqués, selon au moins une instruction, par au moins une image de stimulation. A partir des mouvements oculaires un ou plusieurs paramètres sont calculés et comparés à des valeurs prédéterminées. Cette comparaison permet de déterminer si le sujet humain présente des anomalies oculaires et si oui à quelle pathologie ses anomalies peuvent être associées.

Le système selon l'invention étant totalement automatisée, ne nécessite pas l'intervention d'un spécialiste et encore moins l'intervention d'un collège de spécialistes comme c'est le cas actuellement. Ceci permet de diminuer le temps et les coûts de mise en évidence d'anomalies.

Par ailleurs, la mise en évidence d'anomalies oculaires peut être réalisée en dehors des lieux spécialisés, par exemple chez le sujet humain sain. Ainsi, le système selon l'invention permet d'éviter de mobiliser un IRM ou un laboratoire ainsi que le personnel associé ce qui permet de diminuer encore plus les coûts de mise en évidence d'anomalies.

Enfin, le système selon l'invention permet de déterminer les anomalies des mouvements oculaires plus rapidement que la pratique actuelle. Les essais réalisés montrent que la mise en évidence d'anomalies oculaires sur un sujet humain peut être réalisée en moyenne en 15 minutes contre plusieurs heures actuellement. Une mise en évidence plus rapide des anomalies est très importante pour la santé du sujet humain car cela permet une intervention plus rapide sur le sujet humain.

Par ailleurs, le système selon l'invention réalise la mise en évidence d'anomalies suivant un examen visuel non invasif et donc moins contraignant pour le sujet humain que les interventions invasives actuelles.

La stimulation visuelle peut comprendre un ensemble d'images de stimulation ou un ensemble d'animations visuelles. Ainsi le système selon l'invention peut comprendre :
- des moyens de génération d'au moins une stimulation visuelle d'au moins une cellule visuelle dudit sujet humain, et
- des moyens d'affichage desdites images de stimulation.

Avantageusement, le module d'analyse peut comprendre un sous module réalisant une mise en évidence de saccades.

Par ailleurs, le module d'analyse peut en outre comprendre un sous module de mise en évidence et d'élimination d'artefacts qui permettent de faciliter la lecture et l'analyse automatique du tracé oculomoteur.

Un exemple de mise en évidence de saccades et, un exemple de mise en évidence et d'élimination d'artefacts, sont décrits plus bas.

Le système selon l'invention peut avantageusement comprendre en outre des moyens prévus pour déterminer une probabilité de pathologie chez ledit sujet humain en fonction de la valeur calculée et d'au moins une valeur prédéterminée pour au moins un paramètre. En effet, en fonction de la valeur calculée pour un ou plusieurs paramètres et une ou plusieurs valeurs seuils prédéterminées pour ce(s) paramètre(s), une probabilité de pathologie peut être déterminée pour le sujet humain, par exemple concernant la maladie parkinson.

Selon l'invention, les moyens de capture des mouvements de l'œil du sujet humain peuvent avantageusement comprendre au moins un capteur agencé pour capturer les mouvements oculomoteurs dudit œil pendant que ledit sujet humain est stimulé par lesdites images conformément à au moins une instruction. Un tel capteur peut par exemple être une caméra et plus précisément une caméra infrarouge.

Le système selon l'invention peut en outre comprendre un module de transformation réalisant la transformation des mouvements de l'œil en position sur les moyens d'affichage de stimulation visuelle.

La transformation des mouvements de l'œil en positions peut, dans un premier mode réalisation, être réalisée en temps réel, c'est-à-dire dans un lapse de temps inférieur au temps d'affichage des images de stimulation, par exemple inférieur à 16ms qui est la norme actuelle. Ainsi, lorsque l'œil du sujet humain se déplace sur les moyens d'affichage, la position de l'œil est calculée en temps réel à partir de données fournies par le capteur agencé pour capturer les mouvements oculomoteurs.

Dans un deuxième mode de réalisation, la transformation peut être réalisée *a posteriori.* Dans ce cas les images capturées par la caméra, par exemple une caméra infrarouge, sont dans un premier temps, mémorisées dans des moyens de mémorisation. Dans un deuxième temps, par exemple à la fin de la stimulation de la cellule visuelle du sujet humain, les images capturées sont transformées par le module de transformation en position sur les moyens d'affichage.

Le module d'analyse peut avantageusement être agencé pour déterminer la valeur d'au moins un des paramètres choisi dans la liste suivante :
- latences et vitesses des saccades horizontales (temps de réaction du sujet humain à l'apparition d'un stimulus),
- précision des saccades (distance à la cible),
- nombre de saccades intermédiaires,
- nombre d'erreurs,
- présence d'ondes carrées (saccades isolées qui interrompent la fixation de façon très courte avec retour rapide à cette fixation),
- présence de nystagmus (un mouvement d'oscillation involontaire et saccadé du globe oculaire,
- gain de la poursuite : lors de la poursuite oculaire, le glissement de l'image de la cible visuelle sur la rétine induit un mouvement oculaire de même amplitude pour conserver cette projection sur la macula,
- déphasage de la poursuite,
- qualité de la poursuite.

Cette liste n'est bien sûr pas exhaustive.

On définit une saccade comme suit : quand une nouvelle image est sélectionnée, les mouvements oculaires rapides ou saccades, permettent de changer de point de fixation.

Les valeurs de ces paramètres, ainsi que les valeurs d'autres paramètres, peuvent être déterminées par exemple par étude des positions de l'œil du sujet humain et de la ou les images de stimulation ou encore celle(s) visée(s) en fonction des images de stimulation et des consignes associées à ces images de stimulation. Dans un exemple particulier nullement limitatif, le sujet humain peut recevoir comme consigne de regarder une position opposée à un point affiché par les moyens d'affichage.

Les moyens d'affichage peuvent comprendre éventuellement un écran tactile sur lequel un sujet humain est amené à pointer une position ou à participer à toute autre interaction en fonction de consignes données au préalable.

Le système selon l'invention peut en outre comprendre des moyens de mémorisation des mouvements oculaires et/ou des valeurs des paramètres calculées reliées au module d'analyse et/ou au module de transformation.

Le système selon l'invention peut en outre comprendre des moyens d'affichage des résultats des tests, ainsi que des moyens d'écriture des résultats sur un support.

Dans un mode de réalisation particulier, le système selon l'invention peut avantageusement se présenter sous la forme d'un ensemble monobloc portable. Le système selon l'invention peut se présenter sous la forme, par exemple, de jumelles, de lunettes ou de monture de lunettes, pouvant ainsi être aisément porté par le sujet humain lui-même.

Dans un autre mode de réalisation, le système selon l'invention peut se présenter sous la forme par exemple d'un chariot déplaçable sur des moyens de roulement. Dans le cadre par exemple d'une utilisation en milieu médical.

Le système selon l'invention peut en outre comprendre des moyens de mémorisation pour mémoriser et conserver les données obtenues et éventuellement des moyens d'analyse comparative des données recueillies.

Le système selon l'invention peut en outre comprendre des moyens d'affichage permettant à un opérateur ou un spécialiste d'afficher visuellement les résultats relatifs à plusieurs tests réalisés dans le temps pour un même sujet ou les résultats relatifs à plusieurs tests réalisés sur des sujets différents en vue de réaliser une comparaison de ces résultats.

Le système selon l'invention peut se présenter sous la forme d'une plateforme technologique comprenant des moyens d'affichage.

Selon un autre aspect de l'invention, il est proposé un procédé de mise en évidence d'anomalies oculomotrices chez un sujet humain, ledit procédé de mise en évidence comprenant les étapes suivantes :
- capture, par des moyens de capture, des mouvements de l'œil dudit sujet humain pendant que ledit sujet humain est stimulé par au moins une image conformément à au moins une instruction, lesdits moyens de capture fournissant un signal de capture,
- analyse dudit signal de capture pour calculer la valeur d'au moins un paramètre prédéterminé, et
- détermination d'une anomalie en fonction de ladite valeur calculée à au moins une valeur prédéterminée pour chacun des paramètres.

Le procédé selon l'invention peut en outre comprendre les étapes suivantes :
- génération d'au moins une image de stimulation d'au moins une cellule visuelle dudit sujet humain, et
- affichage de ladite image de stimulation sur des moyens d'affichage.

Le procédé selon l'invention peut avantageusement comprendre une étape de transformation des mouvements de l'œil en position sur les moyens d'affichage des images de stimulation. Cette étape de transformation peut être réalisée en temps réel en même temps que la capture.

Par ailleurs, l'étape d'analyse peut avantageusement comprendre une mise en évidence d'au moins une saccade dans le signal de capture.

L'étape de capture peut aussi comprendre une mise en évidence d'au moins un artefact dans le signal de capture et une élimination dudit artefact détecté de manière à améliorer l'analyse du signal et donc la mise en évidence d'anomalies oculomotrices.

D'autres avantages et caractéristiques de l'invention apparaîtront à l'examen de la description détaillée d'un mode de mise en œuvre nullement limitatif, et des dessins annexés sur lesquels :
- la figure 1 est une représentation schématique d'un exemple de réalisation du système selon l'invention,
- la figure 2 est une représentation schématique d'un exemple d'un module d'analyse selon l'invention, et
- la figure 3 est un exemple d'un signal de capture obtenu avec le système selon l'invention.

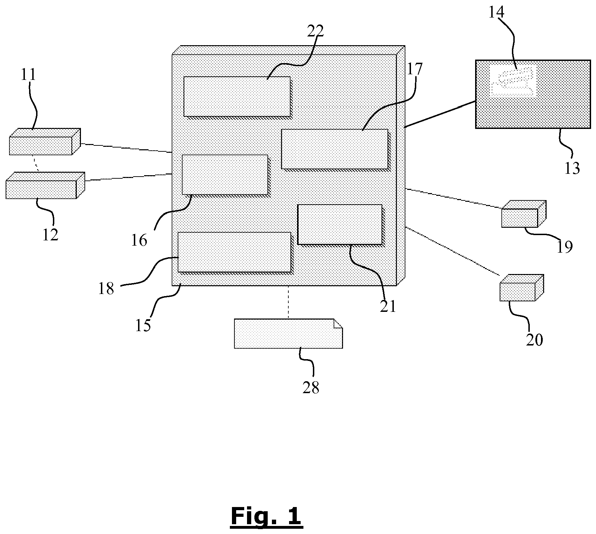

La figure 1 est une représentation schématique d'un exemple de réalisation d'un système selon l'invention.

Le système représenté en figure 1 comprend un écran 11 pour l'affichage images de stimulations (stimuli ou tests). Cet écran peut être un écran plat LCD affichant une résolution de 1920x1200 pixels pour un format 16/9 et de dimension 22 pouces avec une latence d'affichage d'au plus 2ms.

Le système peut en outre comprendre un écran 12 permettant le suivi des tests par un praticien. Cet écran 12 peut être un écran plat LCD capable d'afficher une résolution par exemple de 1920x1200 pixels.

Le système comprend en outre un dispositif 13 de capture des mouvements oculomoteurs. Ce dispositif peut comprendre un moyen de capture et d'enregistrement et de conservation des données (non représentés). Un tel dispositif 13 peut être un dispositif monoculaire ou binoculaire.

Le dispositif 13 de capture des mouvements décrit ici comprend une caméra 14 qui capture et enregistre les mouvements oculomoteurs.

Les écrans 11 et 12 et le dispositif 13 de capture des mouvements oculomoteurs du sujet humain sont reliés à une unité centrale 15 soit de manière filaire soit par une connexion sans fil. L'unité centrale comprend un module 16 de génération des stimuli, un module 17 de transformation des mouvements oculaires en position sur l'écran 11 d'affichage des stimuli et un module 18 d'analyse des mouvements oculaires.

L'unité centrale 15 peut avoir les caractéristiques suivantes :
- processeur,
- Mémoire RAM : Supérieure ou égale à 2GO,
- Carte d'acquisition pour l'interface avec le dispositif 13, et
- Carte graphique : modèle standard ayant des performances au moins égales à celles d'une NVidia quadro fx 4100.

Le système peut en outre comprendre des moyens tels qu'un clavier 19 pour la saisie de données et d'une souris 20 pour effectuer une sélection d'un test de stimuli par une pluralité de tests.

Le système selon l'invention comprend en outre des moyens de mémorisation 21 des tests de stimuli pouvant être sélectionné à l'aide par exemple de la souris 20, le cas échant des moyens pour une analyse comparative avec d'autres signaux par exemple des signaux d'un sujet humain sain.

Par ailleurs, le système représenté en figure 1 comprend en outre un module 22 d'aide au diagnostic permettant de déterminer une probabilité pour une pathologie et de fournir une liste de pathologies classées selon leur probabilité en fonction des résultats des tests fournis par le module d'analyse 18.

Le système peut être relié à un autre appareil 23 directement ou au travers d'un réseau de communications tel que, par exemple, Internet. L'appareil 23 peut être une base de données ou tout autre appareil médical.

Les tests oculaires sont gérés à partir de l'unité centrale 15. Ils sont générés par le module de génération 16 et sont affichés sur l'écran 11. Le sujet humain regarde ces tests avec des instructions pendant que la caméra 14 enregistre les mouvements oculomoteurs du sujet humain. Les mouvements oculomoteurs sont instantanément transformés en position de l'œil sur l'écran 11 par le module 17. Le module 17 transforme le signal de capture fourni par le dispositif de capture 13 et fourni à son tour un « signal position » indiquant à tout moment les positions de l'œil sur l'écran 11. Ce signal de capture est enregistré par l'unité centrale dans les moyens de mémorisation 21. A la fin des tests, les enregistrements sont automatiquement analysés par le module d'analyse 18 et peuvent ensuite être interprétés par le module d'interprétation et d'aide au diagnostic 22.

Le module 16 de génération des stimuli comprend une pluralité de tests de stimuli visuels. Ce module 16 est en outre adapté pour la conception et la mémorisation de nouveaux tests par un praticien.

L'analyse des mouvements oculaires est effectuée de manière automatique et supervisée par le module d'analyse 18.

Les paramètres ainsi mesurés peuvent ensuite être interprétés par le module d'aide au diagnostic 22 qui peut comprendre de l'intelligence artificielle qui proposera un indice de probabilité pour classer la ou les maladie(s) du sujet humain ou directement par un spécialiste.

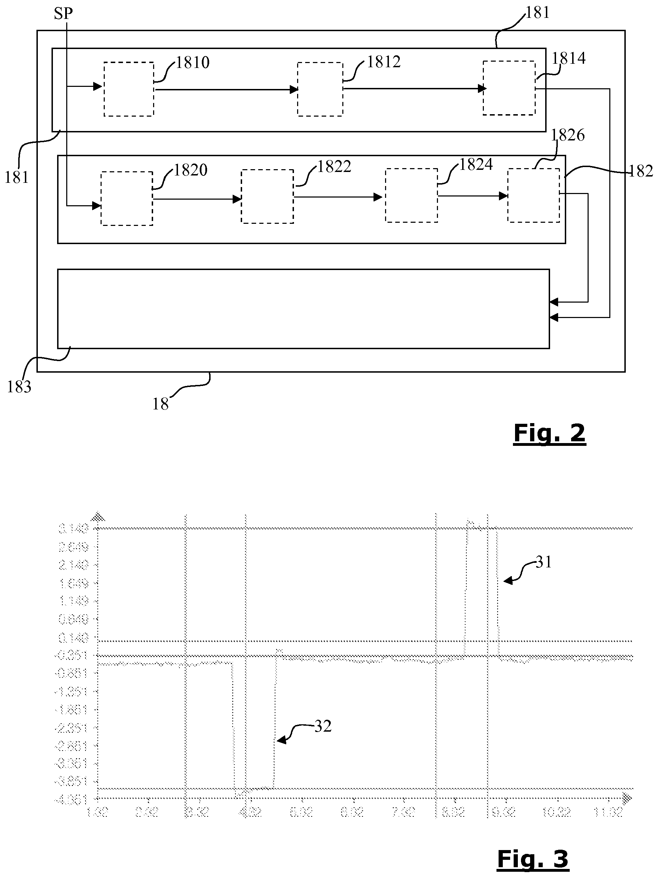

La figure 2 est une représentation schématique d'un exemple de module d'analyse 18.

Le module d'analyse 18 comprend un sous module de mise en évidence des saccades 181. Ce module de mise en évidence des saccades réalise :
- une étape 1810 de traitement préliminaire. Le traitement préliminaire comprend :
   ∘ un filtrage gaussien du signal position SP avec un filtre gaussien de largeur de filtre paramétrable par l'utilisateur, puis
   ∘ une dérivation première du signal obtenu par des moyens de calcul.
- une étape 1812 de mise en évidence de saccades « négatives », i.e. vers la gauche en horizontal, vers le bas en vertical. La mise en évidence des saccades négatives est réalisée de la manière suivante. Tant que la dérivée du signal est négative, on somme les valeurs des échantillons de cette dérivée. Si la vitesse est inférieure à l'opposé de la valeur du seuil de vitesse (paramétrable par l'utilisateur, par défaut : V<-30°/s) et si la somme précédemment calculée dépasse la valeur du seuil d'amplitude (paramétrable par l'utilisateur, par défaut : A>5°) alors des barres de mise en évidence ou de détection sont positionnées sur le premier échantillon et le dernier.
- une étape 1814 de mise en évidence de saccades « positives » (vers la droite en horizontal, vers le haut en vertical). La mise en évidence des saccades positives est réalisée de la manière suivante. Tant que la dérivée est positive, on somme les valeurs des échantillons du signal dérivé. Si la vitesse est supérieure à la valeur du seuil de vitesse (paramétrable par l'utilisateur, par défaut : V>30°/s), et si la somme précédemment calculée dépasse la valeur du seuil d'amplitude (paramétrable par l'utilisateur, par défaut : A>5°), alors des barres de mise en évidence sont positionnées sur le premier échantillon et le dernier

Le module d'analyse 18 comprend en outre un sous module de mise en évidence et de suppression des artefacts 182. Ce module 182 de mise en évidence des artefacts réalise :
- une étape 1820 de traitement préliminaire. Le traitement préliminaire comprend :
   ∘ un filtrage gaussien du signal position SP par un filtre Gaussien de largeur de filtre sigma = 1, et
   ∘ une dérivation première du signal obtenu par des moyens de calcul.
- une étape 1822 de mise en évidence d'artefacts « négatifs », vers la gauche en horizontal, vers le bas en vertical. La mise en évidence des artefacts négatifs est réalisée de la manière suivante. Tant que la dérivée est négative, on somme les valeurs des échantillons du signal dérivé. Si la vitesse est inférieure à un seuil fixé par l'utilisateur (-500°/s par défaut) et si la somme précédemment calculée dépasse 5° et si la durée séparant le premier échantillon du dernier est inférieure à 200 ms, alors on positionne des barres de mise en évidence sur le premier échantillon et le dernier.
- une étape 1824 de mise en évidence d'artefacts « positifs », vers la droite en horizontal, vers le haut en vertical. La mise en évidence des artefacts positifs est réalisée de la manière suivante. Tant que la dérivée est positive, on somme les valeurs des échantillons du signal dérivé. Si la vitesse est supérieure à un seuil fixé par l'utilisateur (500°/s par défaut) et si la somme précédemment calculée dépasse 5° et si la durée séparant le premier échantillon du dernier est inférieure à 200 ms alors on positionne des barres de mise en évidence sur le premier échantillon et le dernier.
- une étape 1826 de suppression des artefacts. La suppression des artefacts est réalisée par récupération des deux échantillons correspondant au début et à la fin de l'artefact détecté. Soient A et B les points correspondant à ces échantillons. La droite (AB) a pour équation : y = mx + p) :
   - Calcul des paramètres « m » et « p » de l'équation de la droite (AB)
   - Recopie des échantillons précédant le point A.
   - Interpolation linéaire suivant la droite (AB) entre les points A et B en conservant la même fréquence d'échantillonnage que le celle du signal original.
   - Recopie des échantillons suivant le point B jusqu'à la fin du signal.

Enfin, le module d'analyse comprend un sous module 183 réalisant le calcul des paramètres oculomoteurs en fonction des données fournies par le sous module de mise en évidence des saccades 181 et le sous module de mise en évidence et de suppression des artefacts 182, pour différentes types de tests.

Bien entendu, la mise en évidence des artefacts et des saccades peut être réalisée par un seul module. Dans ce cas, les artefacts sont détectés dans le signal de position SP puis supprimés, avant de détecter les saccades.

Les différents types de tests sont :
- Gap,
- Antisaccades,
- Saccades verticales,
- Poursuite lisse,

Les paramètres mesurés pour ces différents types de tests par le sous module 183 de calcul des paramètres sont les suivants :
- latence moyenne en ms (temps de déclenchement de la saccade après l'apparition du stimulus visuel)
- vitesse moyenne en °/s des saccades
- vitesse maximum en °/s des saccades
- pourcentage d'erreurs (pour les antisaccades)

### Latence moyenne

La latence correspond à la durée en ms entre l'événement (ex : cible positionnée à droite) et la réponse du sujet humain (barre de début de mise en évidence de saccade).

La latence moyenne est calculée pour les saccades valides :
- Gap : une saccade valide correspond à une saccade où le sujet humain regarde d'abord du côté de la cible (on distingue latence moyenne à droite et latence moyenne à gauche)
- Antisaccades : une saccade valide correspond à une saccade où le sujet humain regarde d'abord du côté opposé à la cible (on distingue latence moyenne des antisaccades cible à droite et latence moyenne des antisaccades cible à gauche)
- Saccades verticales : une saccade valide correspond à une saccade où le sujet humain regarde d'abord du côté de la cible (on distingue latence moyenne vers le haut et latence moyenne vers le bas)

### Vitesse moyenne

La vitesse moyenne est mesurée entre la barre de mise en évidence de début de saccade et la barre de mise en évidence de fin de saccade. La vitesse moyenne est calculée pour les latences valides. On distingue également vitesse moyenne à gauche et à droite (gap et antisaccades) et vers le haut et vers le bas (saccades verticales).

### Pourcentage d'erreurs

Pour les antisaccades, si le sujet humain regarde en priorité dans la direction de la cible, une erreur est comptabilisée.

On distingue pourcentage d'erreurs des antisaccades cible à droite et cible à gauche.

La figure 3 est un exemple de signal position obtenu grâce au procédé et au système selon l'invention. Sur cette figure, on peut observer deux saccades 31 et 32.

Enfin, en fonction des données fournies par le module d'analyse 18 le module 22 d'aide au diagnostic détermine une probabilité pour chaque pathologie et fournit une liste de pathologies classées selon leur probabilité en fonction des résultats des tests.

L'ensemble des pathologies pouvant être détectées avec l'invention, et pour lesquelles l'efficacité et le suivi des soins peut être évaluée, sont les pathologies neurologiques , psychiatriques et neurodéveloppementales par exemple les syndromes parkinsoniens, la maladie d'Alzheimer, la maladie de Creutzfeld-Jacob, les démences à corps de Lewy, les intoxications, les syndromes de Gilles de la Tourette, la schizophrénie, les syndromes bipolaires, les traumas crâniens, la dyslexie, la dyspraxie etc...

Le système selon l'invention permet de passer un examen visuel et d'enregistrer les mouvements oculaires. Le sujet humain regarde des images de stimulation avec des instructions pendant que des moyens de capture enregistrent ses mouvements oculomoteurs. Les mouvements sont instantanément transformés en position de la cellule visuelle sur l'écran et enregistrés par l'ordinateur de contrôle. La mise en évidence automatique supervisée des saccades et de certains paramètres d'oculomotricité, tels que le temps de latence, la vitesse des mouvements oculaires, la précision, et les erreurs, sont ensuite analysés par un logiciel d'intelligence artificielle qui propose alors un indice de probabilité pour classer la pathologie du patient.

Le présent système constitue un test compagnon lors d'une remédiation.

Bien sûr, l'invention n'est pas limitée aux exemples qui viennent d'être décrits et de nombreux aménagements peuvent être apportés à ces exemples sans sortir du cadre de l'invention.

## Revendications

1. Système de mise en évidence d'anomalies oculomotrices chez un sujet humain, ledit système comprenant :
- des moyens de capture (13) des mouvements de l'œil dudit sujet humain pendant que ledit sujet humain est stimulé par au moins une image conformément à au moins une instruction, lesdits moyens de capture (13,14) fournissant un signal de capture,
- un module d'analyse (18) pour calculer la valeur d'au moins un paramètre prédéterminé par analyse dudit signal de capture, et
- des moyens (22) de détermination d'une anomalie en fonction de ladite valeur calculée et au moins une valeur prédéterminée pour chacun des paramètres,
- des moyens de génération (16) d'au moins une stimulation visuelle d'au moins un œil dudit sujet humain,
- des moyens d'affichage (11) desdites images de stimulation, et
- un module (17) de transformation pour transformer les mouvements de l'œil en position sur les moyens d'affichage (11) des images de stimulation.
système dans lequel les moyens de capture (13) comportent une caméra (14) qui capture et enregistre les mouvements oculomoteurs et les moyens d'affichage comportent un écran (11), l'écran (11) et le dispositif (13) de capture des mouvements oculomoteurs dudit sujet humain sont reliés à une unité centrale (15) soit de manière filaire soit par une connexion sans fil,
les images de stimulation comportant des tests oculaires générés par le module de génération (16) de l'unité centrale (15) et affichés sur l'écran (11),
le module de transformation (17) étant configuré pour fournir un signal position (SP) indiquant à tout moment les positions de l'œil sur l'écran (11),
le système comportant des moyens de mémorisation (21) configurés pour enregistrer le signal de capture,
le module d'analyse (18) étant configuré pour analyser automatiquement les enregistrements à la fin des tests.

2. Système selon la revendication 1, comprenant un module (22) d'aide au diagnostic permettant de déterminer une probabilité pour une pathologie et de fournir une liste de pathologies classées selon leur probabilité en fonction des résultats des tests fournis par le module d'analyse (18).

3. Système selon la revendication 1, **caractérisé en ce qu'**il comprend en outre des moyens de comparaison d'enregistrement par rapport aux enregistrements d'un témoin.

4. Système selon l'une des revendications 1 à 3, **caractérisé en ce que** le module d'analyse (18) comprend :
un sous module (181) réalisant une mise en évidence de saccades, le module d'analyse (18) comprenant un sous-module de mise en évidence des saccades (181) configuré pour réaliser:
- une étape (1810) de traitement préliminaire comportant un filtrage gaussien du signal position (SP) avec un filtre gaussien de largeur de filtre paramétrable par l'utilisateur, puis une dérivation première du signal obtenu par des moyens de calcul
- une étape (1812) de mise en évidence de saccades, vers la gauche en horizontal, vers le bas en vertical, comportant l'étape consistant à sommer les valeurs des échantillons de cette dérivée tant que la dérivée du signal est négative, et l'étape consistant à positionner des barres de mise en évidence ou de détection sur le premier échantillon et le dernier si la vitesse est inférieure à l'opposé d'une valeur du seuil de vitesse paramétrable par l'utilisateur et si la somme précédemment calculée dépasse une valeur du seuil d'amplitude paramétrable par l'utilisateur,
- une étape (1814) de mise en évidence de saccades positives, vers la droite en horizontal, vers le haut en vertical, comportant l'étape consistant à sommer les valeurs des échantillons du signal dérivé tant que la dérivée est positive, et l'étape consistant à positionner des barres de mise en évidence sur le premier échantillon et le dernier si la vitesse est supérieure à une valeur du seuil de vitesse paramétrable par l'utilisateur, et si la somme précédemment calculée dépasse une valeur du seuil d'amplitude paramétrable par l'utilisateur,
un sous module (182) réalisant une mise en évidence et une élimination d'artefacts, ce module (182) de mise en évidence des artefacts étant configuré pour réaliser:
- une étape (1820) de traitement préliminaire comportant un filtrage gaussien du signal position (SP) par un filtre Gaussien de largeur de filtre sigma=1, et une dérivation première du signal obtenu par des moyens de calcul,
- une étape (1822) de mise en évidence d'artefacts négatifs, vers la gauche en horizontal, vers le bas en vertical, comportant l'étape consistant à sommer les valeurs des échantillons du signal dérivé tant que la dérivée est négative et l'étape consistant à positionner des barres de mise en évidence sur le premier échantillon et le dernier si la vitesse est inférieure à un seuil fixé par l'utilisateur et si la somme précédemment calculée dépasse 5° et si la durée séparant le premier échantillon du dernier est inférieure à 200 ms,
- une étape (1824) de mise en évidence d'artefacts positifs, vers la droite en horizontal, vers le haut en vertical, comportant l'étape consistant à sommer les valeurs des échantillons du signal dérivé tant que la dérivée est positive, et l'étape consistant à positionner des barres de mise en évidence sur le premier échantillon et le dernier si la vitesse est supérieure à un seuil fixé par l'utilisateur (500°/s par défaut) et si la somme précédemment calculée dépasse 5° et si la durée séparant le premier échantillon du dernier est inférieure à 200 ms
- une étape (1826) de suppression des artefacts comportant la récupération des deux échantillons correspondant au début et à la fin de l'artefact détecté et pour les points A et B correspondant à ces échantillons et la droite (AB) ayant pour équation : y = mx + p) :
• le calcul des paramètres « m » et « p » de l'équation de la droite (AB),
• la recopie des échantillons précédant le point A,
• l'interpolation linéaire suivant la droite (AB) entre les points A et B en conservant la même fréquence d'échantillonnage que le celle du signal original, et
• la recopie des échantillons suivant le point B jusqu'à la fin du signal, et
un sous-module (183) réalisant le calcul des paramètres oculomoteurs en fonction des données fournies par le sous module de mise en évidence des saccades (181) et le sous module de mise en évidence et de suppression des artefacts (182), pour différents types de tests.

5. Système selon la revendication 4, la mise en évidence des artefacts et des saccades étant opérée par un seul module, les artefacts étant détectés dans le signal de position (SP) puis supprimés, avant de détecter les saccades.

6. Systèmes selon la revendication 4 ou 5, **caractérisé en ce que** le module d'analyse (18) est agencé pour déterminer la valeur d'au moins un des paramètres choisis dans la liste suivante :
- temps de latence,
- vitesse de mouvements oculaires, et
- la précision des mouvements oculaires,
- le pourcentage d'erreur des saccades (saccades invalides).

7. Système selon l'une quelconque des revendications précédentes, **caractérisé en ce qu'**il comprend en outre des moyens (22) prévus pour déterminer une probabilité de pathologie chez ledit sujet humain en fonction de la valeur calculée et d'au moins une valeur prédéterminée pour au moins un paramètre.

8. Plateforme technologique comprenant un système selon l'une quelconque des revendications précédentes et au moins un moyen d'affichage.

9. Système selon les revendications 1 à 7 constituant un test compagnon pour remédiation.

## Patentansprüche

1. System zum Nachweis von okulomotorischen Auffälligkeiten bei einem Menschen, das System umfassend:
- Mittel zur Erfassung (13) der Bewegungen des Auges des Menschen, während der Mensch durch mindestens ein Bild entsprechend mindestens einer Anweisung stimuliert wird, wobei die Erfassungsmittel (13, 14) ein Erfassungssignal bereitstellen,
- ein Analysemodul (18) zur Berechnung des Werts mindestens eines vorbestimmten Parameters durch Analyse des Erfassungssignals, und
- Mittel (22) zur Bestimmung einer Auffälligkeit in Abhängigkeit von dem berechneten Wert und mindestens einem vorbestimmten Wert für jeden der Parameter,
- Mittel zur Erzeugung (16) mindestens einer visuellen Stimulation mindestens eines Auges des Menschen,
- Mittel zur Anzeige (11) der Stimulationsbilder, und
- ein Umwandlungsmodul (17) zur Umwandlung der Bewegungen des Auges in eine Position auf den Mitteln zur Anzeige (11) der Stimulationsbilder.
System, wobei die Erfassungsmittel (13) eine Kamera (14) aufweisen, die die okulomotorischen Bewegungen erfasst und aufzeichnet, und die Anzeigemittel einen Bildschirm (11) aufweisen, der Bildschirm (11) und die Vorrichtung (13) zur Erfassung der okulomotorischen Bewegungen des Menschen entweder auf drahtgebundene Weise oder durch eine drahtlose Verbindung mit einer Zentraleinheit (15) verbunden sind, wobei die Stimulationsbilder Augentests aufweisen, die von dem Erzeugungsmodul (16) der Zentraleinheit (15) erzeugt werden und auf dem Bildschirm (11) angezeigt werden, wobei das Umwandlungsmodul (17) dazu ausgebildet ist, ein Positionssignal (SP) bereitzustellen, das zu jeder Zeit die Positionen des Auges auf dem Bildschirm (11) angibt, wobei das System Speichermittel (21) aufweist, die dazu ausgebildet sind, das Erfassungssignal aufzuzeichnen, wobei das Analysemodul (18) dazu ausgebildet ist, die Aufzeichnungen am Ende der Tests automatisch zu analysieren.

2. System nach Anspruch 1, umfassend ein Diagnosehilfsmodul (22), das es gestattet, eine Wahrscheinlichkeit für eine Pathologie zu bestimmen und eine Liste von Pathologien bereitzustellen, die nach ihrer Wahrscheinlichkeit geordnet sind, in Abhängigkeit von den Ergebnissen der Tests, die vom Analysemodul (18) bereitgestellt werden.

3. System nach Anspruch 1, **dadurch gekennzeichnet, dass** es ferner Mittel zum Vergleich von Aufzeichnungen mit Kontrollaufzeichnungen umfasst.

4. System nach einem der Ansprüche 1 bis 3, **dadurch gekennzeichnet, dass** das Analysemodul (18) Folgendes umfasst:
ein Untermodul (181), das einen Nachweis von Sakkaden durchführt, das Analysemodul (18) umfassend ein Untermodul zum Nachweis der Sakkaden (181), das dazu ausgebildet ist, Folgendes durchzuführen:
- einen Schritt (1810) des Vorverarbeitens, aufweisend eine Gauß-Filterung des Positionssignals (SP) mit einem Gauß-Filter einer Filterbreite, die durch den Benutzer parametrierbar ist, dann ein erstes Ableiten des Signals, das durch Rechenmittel erhalten wurde
- einen Schritt (1812) des Nachweisens von Sakkaden horizontal nach links, vertikal nach unten, aufweisend den Schritt, der darin besteht, die Werte der Abtastwerte dieser Ableitung zu summieren, solange die Ableitung des Signals negativ ist, und den Schritt, der darin besteht, Nachweis- oder Detektionsstriche auf dem ersten Abtastwert zu positionieren, und dem letzten, wenn die Geschwindigkeit niedriger als das Negative eines Werts der Geschwindigkeitsschwelle ist, die durch den Benutzer parametrierbar ist, und wenn die zuvor berechnete Summe höher als ein Wert der Amplitudenschwelle ist, die durch den Benutzer parametrierbar ist,
- einen Schritt (1814) des Nachweisens positiver Sakkaden horizontal nach rechts, vertikal nach oben, aufweisend den Schritt, der darin besteht, die Werte der Abtastwerte des abgeleiteten Signals zu summieren, solange die Ableitung positiv ist, und den Schritt, der darin besteht, Nachweisstriche auf dem ersten Abtastwert zu positionieren, und dem letzten, wenn die Geschwindigkeit höher als ein Wert der Geschwindigkeitsschwelle ist, die durch den Benutzer parametrierbar ist, und wenn die zuvor berechnete Summe höher als ein Wert der Amplitudenschwelle ist, die durch den Benutzer parametrierbar ist, ein Untermodul (182), das einen Nachweis und eine Eliminierung von Artefakten vornimmt, wobei dieses Modul (182) zum Nachweis der Artefakte dazu ausgebildet ist, Folgendes durchzuführen:
- einen Schritt (1820) des Vorverarbeitens, aufweisend eine Gauß-Filterung des Positionssignals (SP) mit einem Gauß-Filter einer Filterbreite sigma = 1 und ein erstes Ableiten des Signals, das durch Rechenmittel erhalten wurde,
- einen Schritt (1822) des Nachweisens von negativen Artefakten horizontal nach links, vertikal nach unten, aufweisend den Schritt, der darin besteht, die Werte der Abtastwerte des abgeleiteten Signals zu summieren, solange die Ableitung negativ ist, und den Schritt, der darin besteht, Nachweisstriche auf dem ersten Abtastwert zu positionieren, und dem letzten, wenn die Geschwindigkeit niedriger als eine Schwelle ist, die durch den Benutzer festgelegt wird, und wenn die zuvor berechnete Summe höher als 5° ist und wenn die Dauer, die den erste Abtastwert von dem letzten trennt, niedriger als 200 ms ist,
- einen Schritt (1824) des Nachweisens von positiven Artefakten horizontal nach rechts, vertikal nach oben, aufweisend den Schritt, der darin besteht, die Werte der Abtastwerte des abgeleiteten Signals zu summieren, solange die Ableitung positiv ist, und den Schritt, der darin besteht, Nachweisstriche auf dem ersten Abtastwert zu positionieren, und dem letzten, wenn die Geschwindigkeit höher als eine Schwelle ist, die durch den Benutzer festgelegt wird (500°/s voreingestellt), und wenn die zuvor berechnete Summe höher als 5° ist und wenn die Dauer, die den ersten Abtastwert von dem letzten trennt, niedriger als 200 ms ist
- einen Schritt (1826) des Unterdrückens der Artefakte, aufweisend das Abrufen der zwei Abtastwerte, die dem Anfang und dem Ende des detektierten Artefakts entsprechen, und für die Punkte A und B, die diesen Abtastwerten entsprechen, und die Gerade (AB) die folgende Gleichung aufweist: y = mx + p) :
• das Berechnen der Parameter "m" und "p" der Gleichung der Geraden (AB),
• das Übertragen der Abtastwerte, die dem Punkt A vorausgehen,
• das lineare Interpolieren entsprechend der Geraden (AB) zwischen den Punkten A und B unter Beibehaltung derselben Abtastfrequenz wie jene des Ursprungssignals, und
• das Übertragen der Abtastwerte nach dem Punkt B bis zum Ende des Signals, und ein Untermodul (183), das für verschiedene Testtypen die Berechnung der okulomotorischen Parameter in Abhängigkeit von den Daten vornimmt, die von dem Untermodul zum Nachweis der Sakkaden (181) und dem Untermodul zum Nachweis und zur Unterdrückung der Artefakte (182) bereitgestellt werden.

5. System nach Anspruch 4, wobei das Nachweisen der Artefakte und der Sakkaden durch ein einzelnes Modul vorgenommen wird, wobei die Artefakte in dem Positionssignal (SP) detektiert, dann gelöscht werden, bevor die Sakkaden detektiert werden.

6. Systeme nach Anspruch 4 oder 5, **dadurch gekennzeichnet, dass** das Analysemodul (18) angeordnet ist, den Wert mindestens eines der Parameter zu bestimmen, die aus der folgenden Liste ausgewählt sind:
- Latenzzeit,
- Geschwindigkeit der Augenbewegungen, und
- die Präzision der Augenbewegungen,
- der prozentuale Fehler der Sakkaden (ungültige Sakkaden).

7. System nach einem der vorhergehenden Ansprüche, **dadurch gekennzeichnet, dass** es ferner Mittel (22) umfasst, die dafür vorgesehen sind, eine Pathologiewahrscheinlichkeit bei dem Menschen in Abhängigkeit von dem berechneten Wert und mindestens einem vorbestimmten Wert für mindestens einen Parameter zu bestimmen.

8. Technologieplattform umfassend ein System nach einem der vorhergehenden Ansprüche und mindestens ein Anzeigemittel.

9. System nach den Ansprüchen 1 bis 7, das einen Begleittest für Remediation bildet.

## Claims

1. System for identifying oculomotor anomalies in a human subject, said system comprising:
- capturing means (13) for capturing movements of the eye of said human subject while said human subject is stimulated by at least one image in accordance with at least one instruction, said capturing means (13, 14) supplying a capture signal,
- an analysis module (18) for calculating the value of at least one predetermined parameter by analysing said capture signal, and
- determination means (22) for determining an anomaly on the basis of said calculated value and at least one predetermined value for each of the parameters,
- generation means (16) for generating at least one visual stimulation for at least one eye of said human subject,
- display means (11) for displaying said stimulation images, and
- a transformation module (17) for transforming the movements of the eye in terms of position on the display means (11) for displaying the stimulation images,
in which system the capturing means (13) comprise a camera (14) that captures and records the oculomotor movements and the display means comprise a screen (11), the screen (11) and the capture device (13) for capturing the oculomotor movements of said human subject are connected to a central unit (15) either in a wired manner or through a wireless connection,
the stimulation images comprising ocular tests generated by the generation module (16) of the central unit (15) and displayed on the screen (11),
the transformation module (17) being configured so as to supply a position signal (SP) indicating, at all times, the positions of the eye on the screen (11),
the system comprising storage means (21) configured so as to record the capture signal,
the analysis module (18) being configured so as to automatically analyse the recordings at the end of the tests.

2. System according to Claim 1, comprising a diagnostic assistance module (22) for determining a probability of a pathology and supplying a list of pathologies ranked by their probability on the basis of the results of the tests supplied by the analysis module (18).

3. System according to Claim 1, **characterized in that** it furthermore comprises comparison means for comparing the recordings with recordings from a control subject.

4. System according to one of Claims 1 to 3, **characterized in that** the analysis module (18) comprises:
a submodule (181) that identifies saccades, the analysis module (18) comprising a submodule for identifying saccades (181) configured so as to perform:
- a preliminary processing step (1810) comprising Gaussian filtering of the position signal (SP) with a Gaussian filter having a filter width able to be set by the user, and then first derivation of the signal obtained by calculating means,
- a step (1812) of identifying saccades, horizontally to the left, vertically downwards, comprising the step of summing the values of the samples of this derivative for as long as the derivative of the signal is negative, and the step of positioning identification or detection bars on the first sample and the last if the speed is less than the opposite of a value of the speed threshold able to be set by the user and if the previously calculated sum exceeds a value of the amplitude threshold able to be set by the user,
- a step (1814) of identifying positive saccades, horizontally to the right, vertically upwards, comprising the step of summing the values of the samples of the derived signal for as long as the derivative is positive, and the step of positioning identification bars on the first sample and the last if the speed is greater than a value of the speed threshold able to be set by the user, and if the previously calculated sum exceeds a value of the amplitude threshold able to be set by the user,
a submodule (182) that identifies and eliminates artefacts, this module (182) for identifying artefacts being configured so as to perform:
- a preliminary processing step (1820) comprising Gaussian filtering of the position signal (SP) with a Gaussian filter having a filter width sigma=1, and first derivation of the signal obtained by calculating means,
- a step (1822) of identifying negative artefacts, horizontally to the left, vertically downwards, comprising the step of summing the values of the samples of the derived signal for as long as the derivative is negative, and the step of positioning identification bars on the first sample and the last if the speed is less than a threshold set by the user and if the previously calculated sum exceeds 5° and if the duration between the first sample and the last is less than 200 ms,
- a step (1824) of identifying positive artefacts, horizontally to the right, vertically upwards, comprising the step of summing the values of the samples of the derived signal for as long as the derivative is positive, and the step of positioning identification bars on the first sample and the last if the speed is greater than a threshold set by the user (500°/s by default) and if the previously calculated sum exceeds 5° and if the duration between the first sample and the last is less than 200 ms,
- a step (1826) of eliminating artefacts, comprising recovering the two samples corresponding to the start and to the end of the detected artefact and for points A and B corresponding to these samples and the straight line (AB) having the equation: y = mx + p:
• calculating the parameters "m" and "p" for the equation of the straight line (AB),
• copying the samples before point A,
• linearly interpolating along the line (AB) between points A and B while keeping the same sampling frequency as that of the original signal, and
• copying the samples after point B until the end of the signal, and
a submodule (183) that calculates the oculomotor parameters on the basis of the data supplied by the submodule for identifying saccades (181) and the submodule for identifying and eliminating artefacts (182), for various types of test.

5. System according to Claim 4, the artefacts and the saccades being identified by a single module, the artefacts being detected in the position signal (SP) and then eliminated, before the saccades are detected.

6. System according to Claim 4 or 5, **characterized in that** the analysis module (18) is designed to determine the value of at least one of the parameters chosen from the following list:
- latency time,
- ocular movement speed, and
- the precision of the ocular movements,
- the saccade error percentage (invalid saccades).

7. System according to any one of the preceding claims, **characterized in that** it furthermore comprises means (22) intended to determine a probability of a pathology in said human subject on the basis of the calculated value and of at least one predetermined value for at least one parameter.

8. Technological platform comprising a system according to any one of the preceding claims and at least one display means.

9. System according to Claims 1 to 7, forming a remedial companion test.
